# EUROPÄISCHE PATENTANMELDUNG

(11) **EP 3 123 947 A1**
(43) Veröffentlichungstag der Anmeldung: **01.02.2017**
(21) Anmeldenummer: 15179264.5
(22) Anmeldetag: 31.07.2015
(51) Int. Cl.: A61B 17/02

(54) **DEHNEINRICHTUNG ZUM DEHNEN EINER BAUCHDECKE**

(71) Anmelder: Kantonsspital Basselland, 4101 Bruderholz (CH)
(72) Erfinder: Eucker, Dietmar, 79618 Rheinfelden (DE); Zerz, Andreas, 4059 Basel (CH)
(74) Vertreter: BOVARD AG

(57) **Zusammenfassung**

Eine Dehneinrichtung zum Dehnen einer Bauchdecke (1) an einer Öffnung (2) der Bauchdecke weist Zugelemente (5) mit einem Befestigungsmittel (8; 10) auf, mit dem die Zugelemente an einem Rand der Öffnung (2) lösbar an der Bauchdecke (1) befestigbar ist. Ein Rahmen (3) ist über der Öffnung (2) positionierbar und zum Halten der Zugelemente (5) unter einer Zugkraft (Z) vorgesehen. Die mehreren Zugelemente (5) sind zueinander verteilt um den Rahmen vorgesehen, um die Zugkraft um den Rand der Öffnung verteilt auf die Bauchdecke (1) zu übertragen.

## Beschreibung

Die vorliegende Erfindung betrifft eine Dehneinrichtung zum Dehnen einer Bauchdecke, wie es z. B. für den Verschluss von abdominalen Öffnungen in der Bauchdecke bei rekonstruktiver Bauchdeckenchirurgie erforderlich ist.

In der Bauchdeckenchirurgie stellen z. B. grosse Narbenhernien und Laparostomata nach komplexen Eingriffen mit stark, dehiszenter Bauchmuskulatur eine besondere Herausforderung für Chirurgen dar, die sich mit dem Verschluss offener Bauchwunden mittels rekonstruktiver Bauchdeckenchirurgie beschäftigen. Der Defektverschluss stellt z. B. bei der Narbenhernienchirurgie eines der grössten Probleme dar. Je nach Grösse des Herniendefekts werden verschiedene Techniken angewandt. Diese reichen derzeit meist vom direkten Verschluss von vorderem und hinterem Faszienblatt, der meistens mit etwas Spannung erfolgt und generell mit Netz verstärkt werden kann, über eine laterale Entlastungsinzision in der Bauchmuskulatur, bis hin zur Überbrückung eines Defekts mit Implantaten. Aus der WO 2009/132064 ist zum Beispiel eine Hernienabdeckung bekannt, bei der ein Herniennetz in lösbarer Weise an einem form-flexiblen Rahmen angebracht ist, so dass es in seiner Form angepasst und nach dem Verschluss vom Rahmen gelöst werden kann.

Grundsätzlich besteht die Schwierigkeit, dass eine zurückgewichene Bauchmuskulatur um den Bruchdefekt herum nicht ohne Weiteres durch leichten Zug in ihre Ausgangsposition zurückgebracht werden kann. Bei der Bauchmuskulatur handelt es sich um Skelettmuskulatur, die wie in jedem anderen Körperbereich, retrahiert und nahezu irreversibel verkürzt, wenn sie aus ihrem natürlichen Vordehnungzustand zwischen Ursprung und Ansatz herausgelöst wird oder in verkürztem Zustand ruhiggestellt wird. Somit resultiert daraus eine Kontraktur der Muskulatur. Vor allem bei Mittelliniendefekten ist das Problem der Retraktion bei auseinanderweichenden Rektusmuskeln lateral der Rektusscheide in der verkürzten obliquus Externus-, Internus- und Transversusmuskulatur zu finden. Weiterhin leiden Patienten mit ausgeprägtem Mittelliniendefekt unter der resultierenden Insuffizienz der ventralen Rumpfmuskulatur, die einerseits zu erheblichen funktionellen Einschränkungen führt und andererseits zu einem reflektorisch erhöhtem Tonus der Rumpfmuskulatur, somit auch der residuellen Bauchmuskulatur und Flankemuskulatur, wodurch eine Verkürzung der Muskulatur begünstigt wird.

Da die laterale und zentrale Bauchmuskulatur nicht in einfacher Weise in ihre Ursprungsposition zurückbewegt werden kann, wurden Techniken mit Längengewinn aus der Rektusscheide und der seitlichen Bauchmuskulatur entwickelt. Insbesondere ein Verfahren nach Ramirez hat sich als häufig angewandtes Verfahren durchgesetzt. Durch Trennen von einzelnen Schichten der lateralen Muskulatur können diese gegeneinander verschoben werden. Dabei wird der M. obliquus externus aus seiner anatomischen Position herausbewegt, wodurch ein Längengewinn von 4-5 cm pro Seite erreicht werden kann. Bei anderen Verfahren werden Teile der ventralen Rektusfaszie geschwenkt. Diese Techniken werden nur mehr selten angewandt. Bei diesen Methoden wird die Anatomie der Bauchdecke zwangsläufig verändert, um einen Längengewinn zu erzielen. Daraus resultieren weitere Probleme, wie neue Schwachstellen, grosse Wundflächen oder eine verlängerte Wundheilung, die in Kauf genommen werden müssen.

Aus der DE 11 2011 102 552 T2 ist es bekannt, die Wundränder einer Bauchwunde zueinander zu ziehen. Um Zugkräfte auf die Wundränder ausüben zu können, werden Klett-Bänder an gegenüberliegenden Seiten der Bauchwunde befestigt, wobei gegenüberliegende Klett-Bänder mittels einem Klettverschluss miteinander verbunden werden können. Ein Klett-Band wird an einer inneren Oberfläche der Bauchwand und eine Unterlage auf einer Aussenseite der Bauchwand am Rand der Bauchwunde angeordnet. Das Klett-Band und die Unterlage werden dann durch eine Naht durch die Bauchwand fest miteinander verbunden. Die Klett-Bänder werden unter Zug miteinander verbunden, so dass die gegenüberliegenden Seiten der Bauchwunde aufeinander zu gezogen werden. Dadurch dehnen sich die Muskeln und andere Gewebe unter der statischen Spannung. Mittels der Unterlagen sollen die Zugkräfte über einen grösseren Bereich der Haut ausgebreitet werden und es soll verhindert werden, dass die Kräfte auf einen kleinen Bereich konzentriert sind. Die Zugkräfte, die dabei auf die Bauchdecke einwirken, sind jedoch gering, da sie die Löse-Kraft zum Lösen des Klett-Verschlusses nicht übersteigen dürfen. Zudem besteht bei kleinen Öffnungen das Problem, dass die Überlappung der Klett-Bänder nicht ausreicht, um einen Klett-Verschluss zu bilden, welcher der Zugkraft standhält, die erforderlich ist um eine Dehnung des Gewebes zu erreichen. Ferner werden die Klett-Bänder im Bereich der Wundöffnung angebracht und versperren deren Zugang. Auch wird die Sterilität des Vorgangs beeinträchtigt, da ein Dauerzug am offenen Bauch erforderlich ist. Darüber hinaus ist die Entfernung der Klett-Bänder umständlich und langwierig, so dass sie nicht in einem einzigen operativen Eingriff erfolgen kann.

Es ist eine Aufgabe der vorliegenden Erfindung, eine Dehneinrichtung zum Dehnen einer Bauchdecke an einer Bauchdeckenöffnung zu schaffen, die ein genügendes Dehnen der Bauchdecke um die Bauchdeckenöffnung für eine Rekonstruktion der Bauchdecke ermöglicht, die ein einfaches und schnelles Anbringen an und Lösen von der Bauchdecke erlaubt, die einen individuell anpassbaren Dehneffekt ermöglicht, die eine Behandlung im Bereich der Bauchdeckenöffnung nicht behindert und eine unkomplizierte Anwendung möglichst innerhalb eines einzigen operativen Eingriffs zulässt.

Diese Aufgabe wird von der Erfindung durch eine Dehneinrichtung nach Anspruch 1 gelöst. Vorteilhafte Ausgestaltungen und weitere Ausführungsbeispiele sind in den abhängigen Ansprüchen beschrieben.

Eine Dehneinrichtung zum Dehnen einer Bauchdecke an einer Bauchdeckenöffnung nach der Erfindung weist mehrere Zugelemente mit jeweils einem Befestigungsmittel auf, mit dem das Zugelement an einem Rand der Bauchdeckenöffnung lösbar an der Bauchdecke befestigbar ist. Die Dehneinrichtung weist vorzugweise länglich ausgebildete Zugelemente auf, die um die Öffnung herum zu einander verteilt am Bauchdeckenrand vorgesehen werden. Die Dehneinrichtung weist einen Rahmen auf, der über der Öffnung beabstandet zu dieser positionierbar ist und zum Halten der Zugelemente unter einer Zugkraft vorgesehen ist, wobei mehrere Zugelemente zueinander verteilt um den Rahmen an diesem vorgesehen sind, um die Zugkraft verteilt um den Rand der Öffnung auf die Bauchdecke zu übertragen. Der Rahmen kann Haltemittel aufweisen, mit welchen die Zugelemente unter einer Zugkraft lösbar am Rahmen gehalten werden können. Der Rahmen ist ausreichend steif ausgebildet, um unter Einwirkung der Zugkraft nicht oder nur unwesentlich nachzugeben. Die Zugkraft der Zugelemente greift somit am Bauchdeckenrand an und zieht an der Bauchdecke in Richtung des Verlaufs der Zugelemente. Unter Einwirkung der Zugkraft wird die Bauchdecke gedehnt, so dass eine Behandlung zur Rekonstruktion der Bauchdecke begünstigt wird. Da der Rahmen über der Bauchdeckenöffnung angeordnet ist, verdecken die am Rahmen gehaltenen Zugelemente die Bauchdeckenöffnung kaum und behindern eine Behandlung an oder in der Bauchdeckenöffnung möglichst wenig. Ferner kann die Zugkraft der einzelnen Zugelemente in einfacher Weise angepasst, insbesondere nachgespannt werden.

Der Rahmen kann von einem Gestell über der Bauchdeckenöffnung gehalten werden, wobei das Gestell zum Beispiel an einem Operationstisch befestigt ist. Vorzugsweise ist die Position des Rahmens über der Bauchdeckenöffnung durch ein beweglich ausgestaltetes Gestell einstellbar. Das Gestell kann hierfür Gelenke und längsverschiebbare Streben aufweisen. Ausserdem kann der Rahmen eine verstellbare Geometrie aufweisen, z. B. durch festellbare Gelenke und/oder durch relativ zueinander verstellbare Rahmenteile. Alternativ zur Halterung des Rahmens an einem Gestell kann der Rahmen mit Stützen ausgebildet sein, so dass er auf einer Fläche des Operationstisches oder auf dem Rumpf des Patienten mit der Bauchdeckenöffnung über der Bauchdeckenöffnung abgestützt werden kann. Vorteilhaft sind die Stützen verstellbar und verlängerbar ausgebildet.

In einer Variante weist der Rahmen wenigstens zwei gegenüberliegende Rahmenteile auf, so dass je ein Rahmenteil an gegenüber liegenden Seiten der Bauchdeckenöffnung angeordnet werden kann. Beispielsweise kann der Rahmen viereckig, oval oder parallelogramm-artig geformt sein. In einer bevorzugten Ausführungsform sind die gegenüberliegenden Rahmenteile relativ zueinander beweglich und zwar in einer Richtung auf einander zu oder voneinander weg. Hierfür können diese Rahmenteile z. B. an weiteren Rahmenteilen verschieblich gelagert sein oder sie sind beweglich an einem Gestell angeordnet. Der Rahmen muss daher kein geschlossenes Gebilde sein. Der Raum zwischen den gegenüberliegenden Rahmenteilen ist durch die zueinander beweglichen Rahmenteile variabel einstellbar und kann an die Form einer Bauchdeckenöffnung angepasst werden.

Am Rahmen sind Haltemittel zum Halten der Zugelemente vorgesehen. Vorzugsweise ist für jedes Zugelement ein eigenes Haltemittel am Rahmen vorgesehen. Somit kann an jedem Zugelemente eine individuelle Zugkraft beaufschlagt werden. Es ist aber auch denkbar, dass ein Haltemittel zwei oder mehr Zugelemente gemeinsam hält. Bei dieser Variante kann eine Zugkraft schnell über einen breiten Bereich des Öffnungsrands angelegt werden.

Nach der vorliegenden Erfindung können als Zugelemente z. B. Drahtoder Kabelelemente, Kunststoffschnüre oder dergleichen längliche Elemente verwendet werden. Die Zugelemente werden am einen Ende mit Hilfe der Befestigungsmittel am Rand der Bauchdecke und am anderen Ende mit Hilfe der Haltemittel am Rahmen befestigt. Für die Dehneinrichtung können auch unterschiedliche Arten von Zugelementen verwendet werden. Die Anzahl der Zugelemente richtet sich in der Regel nach der Grösse der Bauchdeckenöffnung. Die Position des Haltelements am Rahmen richtet sich nach der gewünschten Zugrichtung, in der die Zugkraft auf die Bauchdecke wirken soll. Die Zugelemente müssen dabei nicht parallel verlaufen, sondern können sich auch kreuzen.

In einer vorteilhaften Variante der Zugelemente weisen diese zumindest einen elastischen Bereich auf. Vorzugsweise sind sie entlang ihrer Länge überwiegend oder vollständig elastisch ausgebildet. Der elastische Bereich der Zugelemente kann gedehnt werden, so dass er nach einem Spannen für die Zugkraft im Zugelement sorgt. Es ist aber auch möglich Zugelemente zu verwenden, die in sich nicht dehnbar sind und die Zugkraft durch Ziehen am nicht-dehnbaren Zugelement zu erzeugen, beispielsweise durch manuelles Ziehen und Befestigen des Zugelements mit dem Haltemittel am Rahmen unter Zug.

In seiner einfachsten Variante kann das Haltemittel z. B. aus einem Knoten im Zugelement um den Rahmen bestehen, der manuell im Zugelement vorgesehen wird, d. h. das Zugelemente wird unter Zug am Rahmen festgeknotet. In einer vorteilhaften Variante kann als Haltemittel zum Fixieren der Zugelemente am Rahmen z. B. eine Klemmvorrichtung verwendet werden. Die Klemmvorrichtung kann z. B. durch einen V-förmigen Schlitz am oder im Rahmen gebildet werden. Durch seine Dicke wird das längliche Zugelement in bekannter Weise im sich verjüngenden Schlitz festgeklemmt. Es kann auch eine Backenklemme nach Art einer Curryklemme mit zwei beweglichen vorgespannten Klemmbacken verwendet werden, so dass sich das Zugelement durch die darauf wirkende Zugkraft selbst festklemmt. Es ist aber auch möglich ein Haltelement mit einem Halteknopf oder dergleichen vorzusehen.

Die Klemmvorrichtung, der Halteknopf oder dergleichen bilden eine Halterung zum Halten des Zugelements. Das Zugelement kann vorzugsweise wenigstens ein Haltegegenstück aufweisen, das mit der Haltung zusammenwirkt. Das Haltegegenstück kann z. B. durch Verdickungen oder Knoten im Zugelement gebildet sein, die in der Klemmvorrichtung zusätzlich zum Klemmeffekt oder in einem einfachen Schlitz auf Anschlag in Zugrichtung festsitzen. Weiter kann das Haltegegenstück durch Schlaufen oder Schlitze am oder im Zugelement ausgebildet sein, die über den Halteknopf oder dergleichen gestülpt werden. Vorzugsweise sind mehrere Haltegegenstücke entlang der Länge des Zugelements vorgesehen. Das Zugelement kann somit an unterschiedlichen Stellen entlang des Zugelements am Rahmen gehalten werden. Die Länge des Zugelements oder auch die Zugkraft können somit individuell eingestellt werden.

Ein Befestigungsmittel zum Befestigen des Zugelements am Rand der Bauchdecke kann in Form einer Klemme, einer Zange oder eines Hakens vorgesehen sein. Das Befestigungsmittel kann direkt an der Bauchdecke befestigt werden oder es kann an mit der Bauchdecke verbundenen Hilfsmitteln, wie etwa einem Faden oder einer Naht, befestigt sein.

Bei einer Ausführungsform der Dehneinrichtung nach der vorliegenden Erfindung sind erste Zugelemente an einem ersten Rahmenteil und zweite Zugelemente an einem zweiten, dem ersten gegenüberliegenden Rahmenteil derart an den Haltemitteln gehalten, dass die ersten und zweiten Zugelemente gekreuzt zum Bauchdeckenrand verlaufen. Die Zugkraft weist somit eine grosse Komponente parallel zur Bauchdeckenöffnung auf und zieht deshalb einen Randteil, gehalten von ersten Zugelementen, in Richtung eines gegenüberliegenden Randteils, gehalten von zweiten Zugelementen. Die Bauchdeckenöffnung wird somit verkleinert. Vorteilhaft wird der Rahmen dabei nur geringfügig über der Bauchdeckenöffnung angeordnet, um die parallele Komponente der Zugkraft zu vergrössern. Wie vorher erläutert, können die ersten und zweiten Zugelemente auch unter sich gekreuzt sein. Dadurch kann die Zugrichtung zusätzlich auf die individuelle Form einer Bauchdeckenöffnung abgestimmt werden.

Wie bereits geschildert können die Zugelemente durch manuellen Zug am Zugelement gespannt werden. In einer vorteilhaften Variante weist jedoch die Dehneinrichtung am Rahmen wenigstens eine Spannvorrichtung zum Anlegen der Zugkraft an einem Zugelemente auf. Vorzugsweise ist für jedes Zugelement eine eigene Spannvorrichtung vorgesehen und die Spannvorrichtung ist im Haltemittel integriert. Das Haltemittel kann hierfür als Winde mit Rückdrehsperre ausgebildet sein. Durch Betätigung der Winde wird das Zugelement aufgewickelt und eine Zugkraft erzeugt, die über die Befestigungsmittel auf die Bauchdecke übertragen wird. Vorzugsweise ist ein Kraftmesser zur Messung einer von einem Zugelement ausgeübten Zugkraft vorgesehen. Der Kraftmesser kann vorteilhaft in oder an der Spannvorrichtung vorgesehen sein. Zudem wird vorzugsweise ein Kraftbegrenzer zur Begrenzung einer von einem Zugelement ausgeübten Zugkraft vorgesehen. Als Kraftbegrenzer kann z. B. Drehmomentbegrenzer verwendet werden, der eine Betätigung der Spannvorrichtung, wie etwa der Winde, unterbindet, sobald eine vorgegebene Zugkraft am Zugelement erreicht ist. Weiter wird vorzugsweise ein Längenmesser zur Messung einer von einem Zugelement an der Bauchdecke gezogenen Länge vorgesehen.

In einer vorteilhaften Variante der Dehneinrichtung weist wenigstens eines der Zugelemente eine Längenskala entlang seiner Länge auf. Die Skala kann durch Striche, farbige Sektoren und der gleichen am Zugelement ausgebildet sein. Auch können die Haltegegenstücke entlang der Länge des Zugelements als Skala dienen. Mit Hilfe der Skala kann der Dehnungsfortschritt an der Bauchdecke verfolgt werden.

Die Dehneinrichtung zum Dehnen einer Bauchdecke an einer Bauchdeckenöffnung nach der vorliegenden Erfindung erlaubt ein an unterschiedliche Bauchdeckenöffnungen angepasstes Dehnen der Bauchdecke für eine Rekonstruktion der Bauchdecke. Die Dehneinrichtung kann ein einfach und schnell am Rand der Bauchdeckenöffnung angebracht werden. Zudem ist ein schnelles und unkompliziertes Lösen von der Bauchdecke möglich, wie es z. B. bei einem Notfall erforderlich wird. Eine weitere Kontrolle der Wunde wird nicht behindert und der Zugang für einfache Behandlungen an oder in der Bauchdeckenöffnung wird nicht versperrt.

Die Erfindung wurde an Hand mehrerer Ausführungsformen und Varianten dargestellt. Die einzelnen technischen Merkmale einer Ausführungsform können durchaus auch in Kombination mit einer anderen Ausführungsform mit den dargelegten Vorteilen verwendet werden. Die Beschreibung der erfindungsgemässen technischen Merkmale ist daher nicht auf die jeweilige Ausführungsform beschränkt.

Vorteilhafte Ausführungsformen der Erfindung werden im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In den Zeichnungen zeigen:
- Fig. 1a: eine schematische Darstellung einer ersten Ausführungsform einer Dehneinrichtung nach der vorliegenden Erfindung,
- Fig. 1b: eine schematische Darstellung einer zweiten Ausführungsform einer Dehneinrichtung nach der vorliegenden Erfindung,
- Fig. 2a: eine schematische Darstellung eines Rahmens für eine Dehneinrichtung nach der vorliegenden Erfindung in einer ersten Variante,
- Fig. 2b: eine schematische Darstellung eines Rahmens für eine Dehneinrichtung nach der vorliegenden Erfindung in einer zweiten Variante,
- Fig. 2c: eine schematische Darstellung eines Rahmens für eine Dehneinrichtung nach der vorliegenden Erfindung in einer dritten Variante,
- Fig. 3a: eine schematische Darstellung eines Rahmens mit Stützen in einer ersten Variante,
- Fig. 3b: eine schematische Darstellung eines Rahmens mit Stützen in einer zweiten Variante,
- Fig. 4a: eine schematische Darstellung eines herkömmlichen Befestigungsmittels für eine Dehneinrichtung nach der vorliegenden Erfindung zum Befestigen eines Zugelements an einer Bauchdecke in einer ersten Variante,
- Fig. 4b: eine schematische Darstellung eines Befestigungsmittels für eine Dehneinrichtung nach der vorliegenden Erfindung zum Befestigen eines Zugelements an einer Bauchdecke in einer zweiten Variante,
- Fig. 5: eine schematische Darstellung einer Einheit aus Zugelement, Befestigungsmittel und Haltemittel mit Spannvorrichtung für eine Dehneinrichtung nach der vorliegenden Erfindung,
- Fig. 6a: eine schematische Darstellung eines Rahmenteils für einen Rahmen einer Dehneinrichtung nach der vorliegenden Erfindung in einer ersten Variante,
- Fig. 6b: eine schematische Darstellung eines Rahmenteils für einen Rahmen einer Dehneinrichtung nach der vorliegenden Erfindung in einer zweiten Variante,
- Fig. 7a: eine schematische Darstellung eines Rahmenteils mit Haltemittel und darin gehaltenem Zugelement in einer ersten Variante,
- Fig. 7b: eine schematische Darstellung eines Rahmenteils mit Haltemittel und darin gehaltenem Zugelement in einer zweiten Variante,
- Fig. 7c: eine schematische Darstellung eines Rahmenteils mit Haltemittel und darin gehaltenem Zugelement in einer dritten Variante und
- Fig. 8: eine schematische Darstellung einer weiteren Ausführungsform einer Spannvorrichtung für eine Dehneinrichtung nach der vorliegenden Erfindung,
- Fig. 9a: ein schematisches Beispiel für ein Bauchdecken-Distrationssystem für den Einsatz mit einer Dehneinrichtung nach der Erfindung,
- Fig. 9b: ein schematisches Beispiel für ein Bauchdecken-Distrationssystem mit einer Dehneinrichtung nach der ersten Ausführungsform, wie in Figur 1a gezeigt,
- Fig. 9c: ein schematisches Beispiel für ein Bauchdecken-Distrationssystem mit einer Dehneinrichtung nach der zweiten Ausführungsform, wie in Figur 1b gezeigt,
- Fig. 10a: ein erstes schematisches Beispiel einer Halterung für eine erste Variante eines Rahmens einer Dehneinrichtung nach der Erfindung und
- Fig. 10b: ein zweites schematisches Beispiel einer Halterung für eine zweite Variante eines Rahmens einer Dehneinrichtung nach der Erfindung.

Es werden verschiedene Ausführungsbeispiele und Varianten einer Dehneinrichtung zum Dehnen einer Bauchdecke an einer Öffnung der Bauchdecke zur Rekonstruktion der Bauchdecke vorgestellt. Dabei werden vorteilhaft Materialien verwendet, wie sie bereits bei bestehenden Operationsverfahren zum Einsatz kommen und somit keine zusätzlichen Gefahren für den Patienten hervorrufen. Alle verwendeten Materialien sind derart ausgewählt, dass sie mit im medizinischen Bereich üblichen Verfahren sterilisierbar sind.

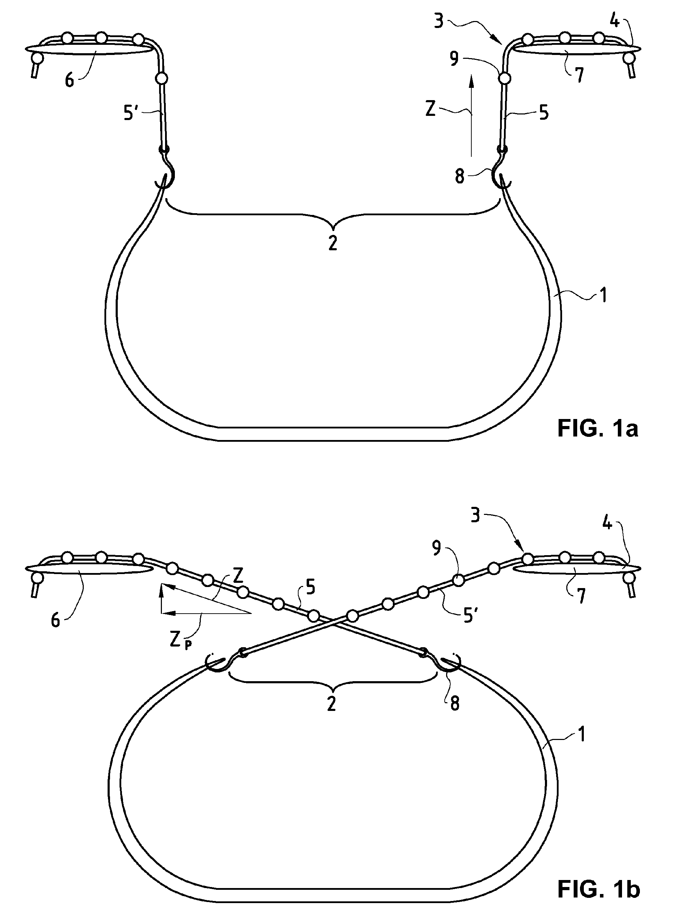

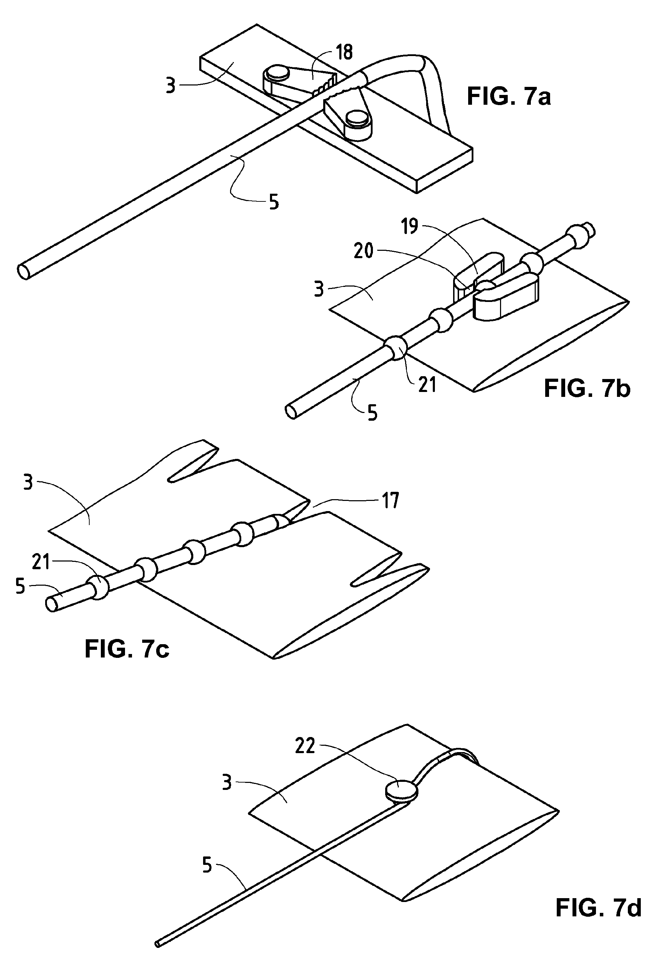

In den Figuren 1 a und 1 b sind eine erste und zweite Ausführungsform einer Dehneinrichtung zum Dehnen einer Bauchdecke 1 an einer Öffnung 2 der Bauchdecke nach der Erfindung gezeigt. Ein Rahmen 3 ist oberhalb der Bauchdeckenöffnung 2 angeordnet und weist Haltemittel 4 zum Halten von Zugelementen 5 auf. Der Rahmen 3 umfasst ein erstes Rahmenteil 6 und ein zweites Rahmenteil 7, das dem ersten Rahmenteil 6 gegenüberliegt. Die Rahmenteile 6 und 7 sind jeweils an gegenüberliegenden Seiten der Bauchdeckenöffnung 2 derart vorgesehen, dass die Öffnung zwischen Ihnen zu liegen kommt. Die Zugelemente 5 weisen an einem Ende Befestigungsmittel auf, die in dieser Variante als Haken 8 ausgebildet sind. Die Haken sind am Rand der Bauchdeckenöffnung in die Bauchdecke 1 eingehakt und sind dadurch fest aber lösbar mit der Bauchdecke verbunden. Am anderen Ende sind die Zugelemente in gespanntem Zustand im Haltemittel 4 gehalten, so dass eine Zugkraft Z auf die Bauchdecke wirkt. Die Zugelemente 5 weisen entlang ihrer Länge mehrere Verdickungen 21 auf, die zum Halten des Zugelements im Haltemittel 4 dienen, wie bei Figur 7c genauer erläutert wird. Der Rahmen 3 wird vorteilhaft in einer Höhe zwischen 5 cm und 30 cm über der Bauchdecke angeordnet, je nach Art und Form der Bauchdeckenöffnung. Vorzugsweise wird der Rahmen 3 in einer Höhe zwischen 15 cm und 25 cm angeordnet. Gute Ergebnisse zur Dehnung der Bauchdecke wurden bei ca. 20 cm erreicht.

In Figur 1 a sind erste Zugelemente 5 (wovon nur eines beispielhaft gezeigt ist) von einer Seite des Bauchdeckenrands zum darüber liegenden zweiten Rahmenteil 7 geführt, das auf der gleichen Seite der Öffnung liegt. Auf der gegenüberliegenden Seite der Öffnung sind zweite Zugelemente 5' von dieser gegenüberliegenden Seite des Bauchdeckenrands zum darüber liegenden ersten Rahmenteil 6 geführt, das ebenfalls auf der gegenüberliegenden Seite liegt. Die ersten Zugelemente 5 werden somit zum auf derselben Seite der Öffnung vorgesehenen zweiten Rahmenteil 7 und die zweiten Zugelemente 5' zum auf der gegenüberliegenden Seite vorgesehenen ersten Rahmenteil 6 geführt ohne die Bauchdeckenöffnung 2 zu überspannen. Die Öffnung 2 bleibt daher frei zugänglich. Die Zugkraft der Zugelemente 5 und 5' verläuft in der Ausführungsform nach Figur 1a überwiegend senkrecht nach oben und dehnt die Bauchdecke am Rand der Öffnung.

In Figur 1 b ist eine andere Ausführungsform der Dehneinrichtung gezeigt, bei der die ersten Zugelemente 5 auf der einen Seite des Rands der Bauchdecke mit den Befestigungsmitteln befestigt sind, aber zum ersten Rahmenteil 6 auf der gegenüberliegenden Seite des Bauchdeckenrands geführt sind. Entsprechend sind die zweiten Zugelemente 5' von der anderen, gegenüberliegenden Seite des Rands der Bauchdecke zum zweiten Rahmenteil 7 geführt. Das heisst, erste Zugelemente 5 sind an einem ersten Rahmenteil 6 und zweite Zugelemente 5' an einem zweiten, dem ersten gegenüberliegenden Rahmenteil 7 derart an den Haltemitteln 4 gehalten, dass die ersten Zugelemente 5 und die zweiten Zugelemente 5' gekreuzt verlaufen. Zwar überspannen bei dieser Ausführungsform die Zugelemente 5 und 5' die Bauchdeckenöffnung. Es bleibt aber zwischen den Zugelementen ausreichend Freiraum, um Zugriff in die Bauchdeckenöffnung zu erlauben. Aufgrund der gekreuzten Führung der Zugelemente 5 und 5' verläuft die Zugkraft in den Zugelemente überwiegend horizontal und somit parallel zur Bauchdecke. Die parallele Komponente Zp der Zugkraft ist in Figur 1 b im Kräftedreieck gezeigt. Die senkrechte Komponente ist offensichtlich deutlich kleiner als die horizontale Komponente Zp. Die sich gegenüberliegenden Seiten des Bauchdeckenrandes werden daher aufeinander zu gezogen, um die Öffnung zu schliessen.

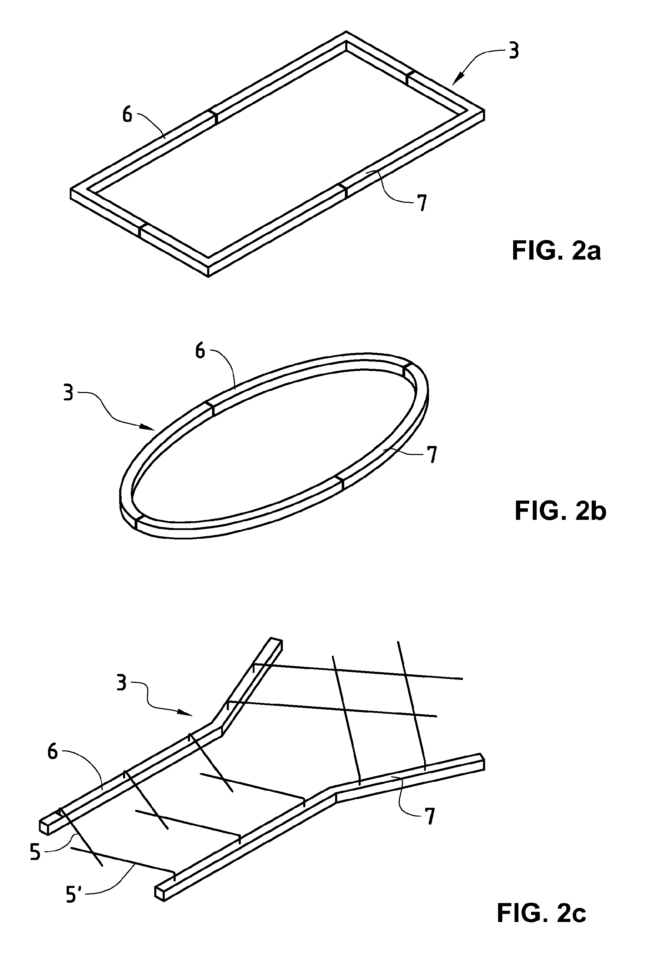

In den Figuren 2a bis 2c sind verschiedene Varianten eines Rahmens für eine Dehneinrichtung nach der Erfindung gezeigt. Figur 2a zeigt einen viereckigen Rahmen 3 und Figur 2b einen ovalen Rahmen 3. Beide Rahmen 3 weisen einen Teilbereich als ersten Rahmenteil 6 und einen gegenüberliegenden Teilbereich als zweiten Rahmenteil 7 auf. Ferner sind diese Rahmen in sich geschlossen. Figur 2c zeigt einen Rahmen 3, der aus zwei einzelnen Teilelementen besteht, die einander gegenüberliegend angeordnet sind. Jedes Element bildet einen der Rahmenteile 6 und 7. Die Rahmenteile 6 und 7 sind in dieser Variante relativ zueinander beweglich, so dass der Zwischenraum an die Form einer Bauchdeckenöffnung angepasst werden kann. Die dargestellten Formen der Rahmen zeigen übliche Rahmengeometrien. Grundsätzlich ist es aber denkbar, die Form des Rahmens individuell an die anatomischen Vorgaben einer Bauchdeckenöffnung anzupassen. Alle diese Rahmen können mittels einem Gestell über der Bauchdecke eines Patienten angeordnet und deren Position eingestellt werden. Das Gestell ist vorteilhaft z. B. an einem Operationstisch befestigt. Als Gestelle können bekannte Systeme mit Kloben- und Klemmsystemen, Flügelschrauben, Rändelrädern, Schnellspannexzentern, etc. verwendet werden.

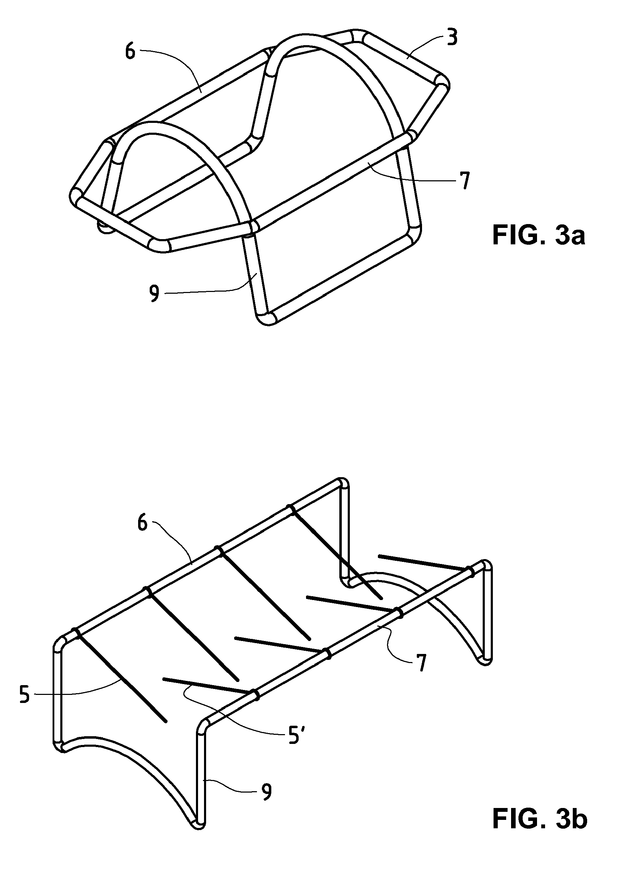

In den Figuren 3a und 3b sind zwei weitere Varianten eines Rahmens 3 für eine Dehneinrichtung nach der Erfindung gezeigt, bei welchen der Rahmen 3 Stützen 9 aufweist, mit welchen der Rahmen 3 über oder auf einem Patienten mit einer offenen Bauchdecke abgestützt werden kann. Es ist somit kein zusätzliches Gestell zum Halten des Rahmens erforderlich. Die einzelnen Elemente des Rahmens können dennoch mittels Gelenken und Schiebeführungen relativ zu einander beweglich vorgesehen sein. Diese Rahmenvarianten sind besonders für eine gekreuzte Führung der Zugelemente 5 und 5' geeignet, wie sie in Figur 1 b dargestellt ist, da die Zugkraft überwiegend durch die horizontale Komponente bestimmt ist und ein Aufpressen der Stützen 9 des Rahmens 3 auf den Patienten vermieden wird. Die Krafteinwirkung auf die Bauchdecke wird durch den gegenwirksamen Zug innerhalb des Rahmens aufgenommen und muss nicht extern abgestützt werden.

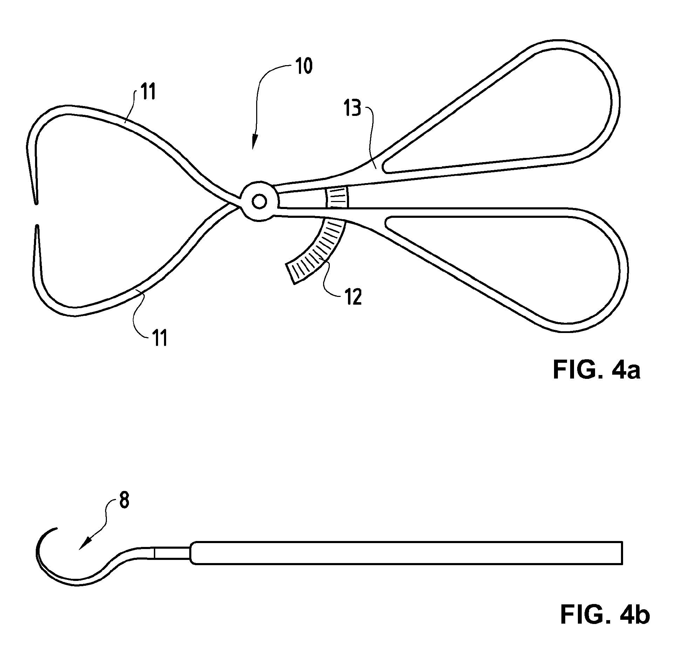

In den Figuren 4a und 4b sind zwei Varianten eines Befestigungsmittels zum Befestigen eines Zugelements an der Bauchdecke für eine Dehneinrichtung nach der Erfindung gezeigt. Die Befestigungsmittel sollen möglichst atraumatisch an der Bauchdecke befestigt werden, müssen die Zugelemente aber sicher an der Bauchdecke halten.

In Figur 4a ist als Befestigungsmittel eine Klemme 10 nach der Art einer Krallenklemme vorgesehen. Die Klemme 10 weist zwei zusammenwirkende spitze Branchen 11 zum Greifen der Bauchdecke und einen Verschlussmechanismus 12 auf, der einem ungewollten Öffnen der Klemme 10 entgegen wirkt. Die Klemme kann auch zwei oder mehr Branchen 11 aufweisen. Die Klemme 10 kann mittels Halmen 13 mit Augen manuell betätigt werden. Der Verschlussmechanismus 12 kann z. B. durch ein Rast-, Klemm-, Schraub- oder Riegelsystem gebildet sein. Als Klemme 10 kann z. B. eine Backhaus- oder Tuchklemme verwendet werden. Mit einer solchen Klemme 10 kann sowohl die Bauchdecke mit allen Schichten gleichzeitig oder z. B. nur Faszie, Muskulatur oder Narbengewebe gefasst werden. Derartige Klemmen können in unterschiedlichen Grössen zur Anwendung kommen. Vorteilhaft können dabei bereits zum Operationsbedarf gehörende Klemmen verwendet werden, da diese bereits den hohen Anforderungen an Operationsutensilien entsprechen. In Figur 4b ist als Befestigungsmittel ein einfacher Haken 8 gezeigt, der durch die Bauchdecke gestochen wird. Die Klemme 10 und der Haken 8 können in üblicher Weise an den Zugelementen befestigt werden, etwa durch Einhängen, Knoten, Verschrauben, Festklemmen, etc. Auch nicht lösbare Befestigungen sind denkbar.

Zur Befestigung der Zugelemente an der Bauchdecke können auch Nähte oder Schlaufen verwendet werden. Zudem können entlang des Bauchdeckenrandes verlaufende Elemente, wie Stäbe, Drähte, etc., verwendet werden, an welchen die Befestigungsmittel angebracht werden, um die vom Zugelement übertragene Kraft in der Bauchdecke zu verteilen. Vorteilhaft wird eine fortlaufende Naht verwendet, die einen kraftaufnehmenden Draht mitumfasst. Die Krafteinwirkung eines Zugelements kann dadurch auf mehrere Nahtpunkte an der Bauchdecke übertragen, d. h. verteilt werden.

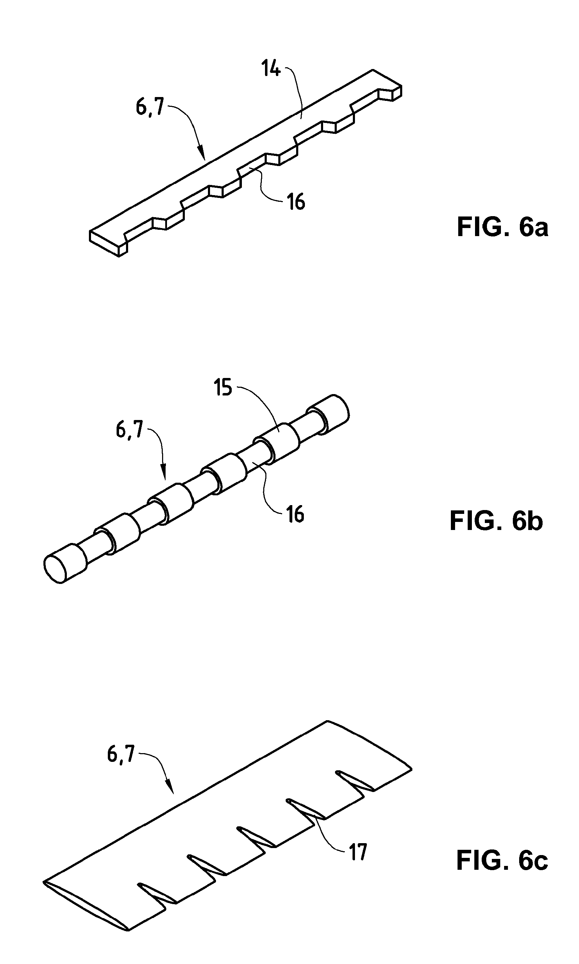

In den Figuren 6a bis 6c sind verschiedene Varianten eines Rahmenteils 6 oder 7 für einen Rahmen einer Dehneinrichtung nach der Erfindung gezeigt. In Figur 6a wird als Rahmenteil eine Schiene 14 mit flachem rechteckigen Querschnitt gezeigt. In Figur 6b wird eine Stange 15 mit rundem Querschnitt verwendet. Sowohl die Schiene 14 als auch die Stange 15 weisen Einkerbungen 16 entlang ihrer Länge auf, die eine Rasterung bilden. Die Rasterung kann ein Verrutschen der am Rahmenteil angebrachten Zugelemente verhindern. Weiter kann sie als Indexierung zur Abstandsbestimmung zwischen mehreren am Rahmenteil angebrachten Zugelementen dienen.

In Figur 6c ist ein flächig ausgebildetes längliches Rahmenteil 6, 7 gezeigt, dass entlang einer Kante mehrere Klemmschlitze 17 aufweist, die eine Halterung zum Halten der Zugelemente bilden. Die Klemmeschlitze 17 können sich verjüngend ausgebildet sein, so dass die Zugelemente durch die Zugkraft eigenständig in einen Klemmsitz gezogen werden.

Als Zugelemente können, wie eingangs erwähnt, Kabel, Drähte, Schnüre, Kunststoffseile, elastische Elemente, wie Gummis, auch mit unterschiedlichen Elastizitätsmodulen, verwendet werden. Auch können Kombinationen daraus verwendet werden. Diese Zugelemente kommen ohne zusätzliche Halteelemente aus und können in einfacher Weise an den vorher beschriebenen Rahmenteilen 6, 7 angeknotet oder eingeklemmt werden.

In den Figuren 7a bis 7d sind verschiedene Varianten von Haltemitteln zum Halten der Zugelemente am Rahmen für eine Dehneinrichtung nach der Erfindung gezeigt. In Figur 7a ist ein Haltemittel in Form einer vorgespannten Backenklemme 18 mit zwei Klemmbacken nach Art einer Curryklemme gezeigt, die ein Zugelement 5 in Form eines Seils zwischen sich aufnehmen und festklemmen. Das Zugelement 5 kann in einfacher weise gespannt werden, indem es durch die Backen gezogen wird. Die Spannkraft bewirkt die Zugkraft an der Bauchdecke und bewirkt eine Selbsthemmung zum Einklemmen des Seils zwischen den Klemmbacken.

In Figur 7b ist ein Haltemittel mit einer Halterung in Form zweier Haltebacken 19 gezeigt, die auf dem Rahmen 3 angebracht sind und das Zugelement 5 zwischen sich aufnehmen können. In Zugrichtung des Zugelements 5 weisen die Haltebacken 19 einen Anschlag 20 auf. An dem Zugelement 5 sind mehrere Verdickungen 21 als Haltegegenstücke entlang der Länge des Zugelements 5 vorgesehen. Wird das Zugelement 5 unter Zugkraft zwischen die Haltebacken 19 eingelegt, wird das Zugelement durch die Zugkraft mit der in der Halterung liegenden Verdickung 21 gegen den Anschlag gepresst und in der Halterung gehalten.

In Figur 7c wird als Halterung ein sich verjüngender Klemmschlitz 17 im Rahmen 3 verwendet, wie in Figur 6c gezeigt. Es wird ein Zugelement 5 mit Verdickungen 21 verwendet, wie bei Figur 7b. Auch hier schlagen die Verdickungen als Haltegegenstücke am Rand der Klemmschlitze 17 an, sobald das Zugelement 5 unter Zugkraft im Klemmschlitz 17 festsitzt. Halterung und Haltegegenstück können gemeinsam das Haltemittel bilden.

In Figur 7d wird als Haltemittel ein von der Oberfläche des Rahmens 3 abstehender Knopf 22 vorgesehen. Ein Zugelement 5 kann in einfacher Weise am Knopf 22 festgebunden werden. Im Falle eines Gummibandes als Zugelement kann dieses um den Knopf 22 gewickelt werden, bis es sich selbsthemmend festzieht. Das Zugelement 5 kann aber auch zusätzliche Haltegegenstücke wie Knopflöcher oder Ösen entlang seiner Länge aufweisen.

Bei den Varianten der Figuren 7a bis 7c und der Variante nach Figur 7d mit zusätzlichen Haltegegenstücken kann in einfacher Weise ein Nachspannen der Zugelemente 5 erfolgen, wenn z. B. die Bauchdecke nach gewisser Zeit nachgegeben und die Zugkraft sich verringert hat. Es muss lediglich am freien Ende des Zugelements gezogen werden, um es aus dem Haltesitz zu lösen, und es kann an verkürzter Position wieder in die Halterung eingelegt werden, bzw. die Selbsthemmung hält das Zugelement in der neuen Position.

Vorteilhaft sind die Halterungen oder Haltemittel am Rahmen verschiebbar angeordnet, um die Position der Zugelemente und die Zugrichtung der Zugkraft einstellen zu können. Hierfür können die Halterungen oder Haltemittel z. B. auf einem Schlitten vorgesehen werden, der in einer Schiene entlang des Rahmens verfahrbar und feststellbar gelagert ist.

Grundsätzlich sind auch andere gängige Haltemechanismen denkbar, wie etwa ein Festschrauben der Zugelemente, diese sind jedoch meist sehr umständlich in der Handhabung oder aufwendig in der Konstruktion.

Das Spannen der Zugelemente kann manuell erfolgen. Die Zugkraft kann allein aufgrund der Elastizität der Bauchdecke entstehen. Die Zugkraft kann auch durch elastische Zugelemente oder Zugelemente mit Federelement aufgebracht werden. Zugelemente die selbst eine Zugkraft erzeugen, können auch dann noch ein Dehnen der Bauchdecke bewirken, wenn die Bauchdecke bereits nachgegeben hat.

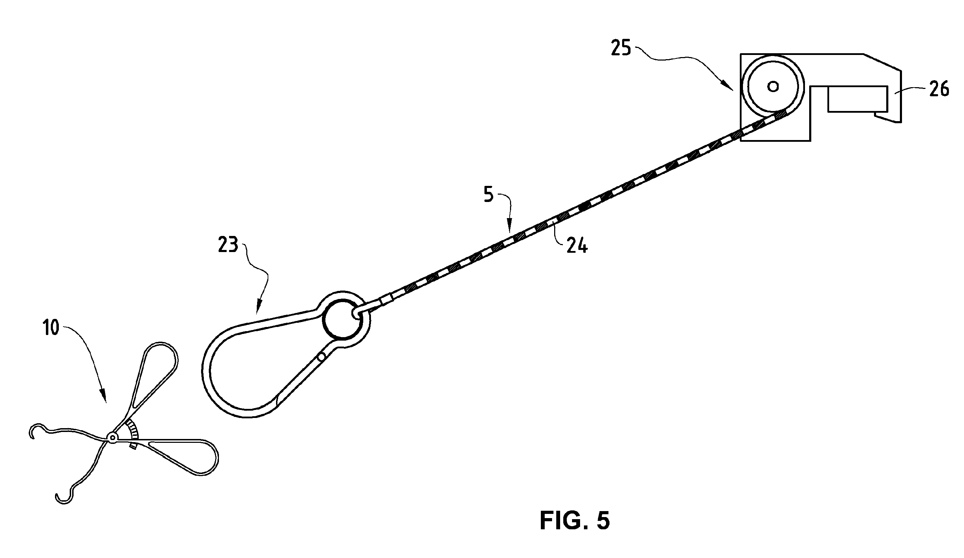

In Figur 5 ist eine Einheit aus Befestigungsmittel in Form einer Klemme 10 in Kombination mit einem Karabiner 23, einem Zugelement 5 mit einer Skala 24 und einer Spannvorrichtung 25 in Form einer Winde mit Rückdrehsperre vorgestellt. Die Spannvorrichtung 25 weist einen Haltearm 26 auf, mit dem sie am Rahmen 3 gehalten werden kann. Der Haltearm 26 kann entlang des Rahmens 3 verschoben werden und an gewünschter Position eingehakt werden. Die Spannvorrichtung kann mit einem Drehhebel betätigt werden, so dass die Winde das Zugelement 5 aufwickelt und eine Zugkraft im Zugelement erzeugt. Weiter kann ein Kraftmesser vorgesehen sein, in der Spannvorrichtung oder als gesondertes Bauteil, der die aufgebaute Zugkraft bestimmt. Damit kann ein Nachlassen der Zugkraft beobachtet werden und eine zu hohe Zugkraft vermieden werden. Vorteilhaft wird weiter ein Kraftbegrenzer vorgesehen, der bei Überschreiten einer vorbestimmten maximalen Zugkraft ein weiteres Spannen des Zugelements unterbindet. Hierfür kann z. B. ein Drehmomentbegrenzer, wie etwa eine Rutschkupplung, in der Spannvorrichtung vorgesehen werden. Eine solche Einheit kann z. B. im Rahmen systematischer Untersuchungen vorteilhaft zur Erfassung der erforderlichen Kräfte eingesetzt werden.

In einer Variante kann die Spannvorrichtung auch motorisiert betrieben werden. Auch kann die Spannvorrichtung statt einer Winde zum Aufwickeln der Zugelemente als Zahngetriebe vorgesehen sein, das in eine Verzahnung am Zugelemente eingreift.

Vorteilhaft wird zudem ein Längenmesser zur Bestimmung der durch die Dehnung erfolgten Verlängerung der Bauchdecke vorgesehen. Dies kann in üblicher Weise durch eine Lasermarkierung erfolgen. Auch kann anhand der Skala 24 eine vom Zugelement zurückgelegte Strecke und damit eine an der Bauchdecke gedehnte Länge bestimmt werden. Es kann auch die Bruchdehiszenz ermittelt werden, um zu bestimmten, ob ein Verschluss der Bauchdeckenöffnung möglich ist oder nicht.

In einer Ausführungsform können der Kraftmesser, der Kraftbegrenzer und der Längenmesser in der Spannvorrichtung integriert sein. Mit Hilfe einer Steuereinheit kann dann die Spannvorrichtung entsprechend vorgegebener Richtwerte für unterschiedliche Arten von Bauchdeckendefekten gesteuert und eine passende Zugkraft eingestellt werden. Auch können unterschiedliche Spannvorrichtungen für unterschiedliche Zugelemente mit Hilfe der Steuereinheit aufeinander abgestimmt werden.

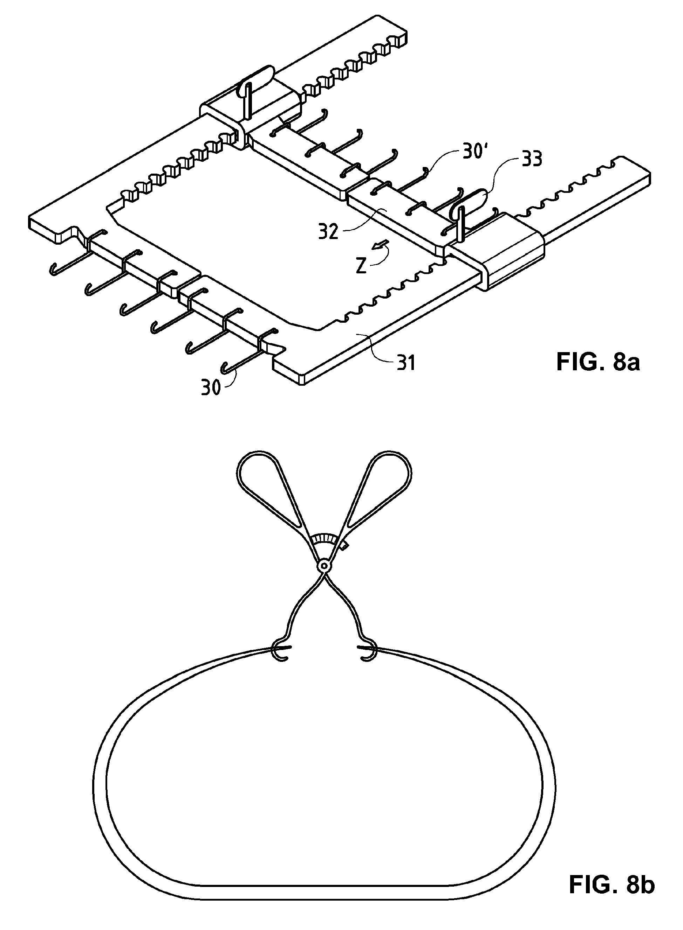

In Figur 8 wird eine weitere Ausführungsform einer Spannvorrichtung schematisch dargestellt. Die Spannvorrichtung kann zwischen Befestigungsmittel und Rahmen an einem Zugelement oder übergreifend an mehreren Zugelementen angeordnet werden. Beispielsweise kann das Zugelement an Haken 30 und 30' befestigt werden. Die Haken 30 werden an einem ersten Zugelementteil eingehakt, das z. B. das Befestigungsmittel zur Befestigung an der Bauchdecke aufweist, und sind an einer Zahnstange 31 befestigt. Die Haken 30' werden an einem zweiten Zugelementteil eingehakt, das z. B. am Rahmen 3 gehalten wird, und sind an einem Träger 32 befestigt. Der Träger 32 umfasst ein Zahnrad, das in die Verzahnung der Zahnstange 31 eingreift. Durch Drehen des Zahnrads z. B. mittels eines Schraubhebels 33 kann das Zahnrad entlang der Zahnstange 31 verfahren werden und nimmt dabei den Träger 32 mit. Dadurch werden die Haken 30' am Träger 32 relativ zu den Haken 30 an der Zahnstange 31 bewegt, so dass in den Zugelementen eine Zugkraft Z erzeugt wird.

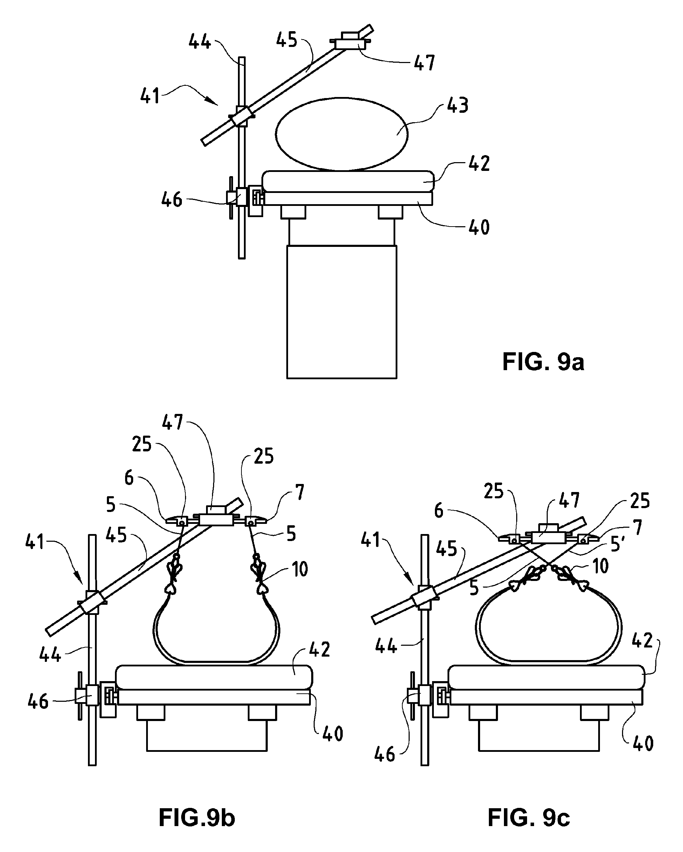

In Figur 9a wird schematisch ein Bauchdecken-Distrationssystem gezeigt, das den Einsatz einer Dehneinrichtung nach der Erfindung unterstützt. Das System umfasst einen Operationstisch 40, der höhenverstellbar ausgebildet ist, und ein verstellbares Traggestell 41. Auf dem Operationstisch 40 ist eine Auflage 42 vorgesehen, auf der ein Patent 43 gelagert werden kann. Das Traggestell 41 umfasst mehrere Streben, wie etwa eine Vertikalstrebe 44 und eine zumindest teilweise horizontal verlaufende Horizontalstrebe 45. Die Vertikalstrebe 44 kann am Operationstisch 40 höhenverstellbar angebracht werden, beispielsweise mittels Kloben 46 an einer Seitenführung des Tisches. Die Horizontalstrebe 45 ist an einem Ende an der Vertikalstrebe 45 angebracht und kann entlang dieser in der Höhe und im Winkel dazu verstellt werden. Am anderen der Horizontalstrebe 45 ist ein Befestigungsmittel 47 angeordnet, an dem die Dehneinrichtung nach der Erfindung z. B. mit dem Rahmen 3 befestigt werden kann und das entlang der Horizontalstrebe 45 verschieblich ist. Grundsätzlich ist es möglich, mehrere Traggestelle 41 am Operationstisch 40 vorzusehen, beispielsweise um besondere Ausgestaltungen der Dehneinrichtung berücksichtigen zu können. Mittels des Traggestells oder der Traggestelle 41 kann die Dehneinrichtung individuell relativ zur Bauchdeckenöffnung 2 positioniert werden.

In den Figuren 9b und 9c sind Varianten der ersten und zweiten Ausführungsform einer Dehneinrichtung nach der vorliegenden Erfindung analog den Figuren 1 a und 1 b dargestellt. Für eine bessere Übersichtlichkeit wurde auf die Darstellung eines Patienten verzichtet. Die Dehneinrichtung umfasst erstes und ein zweites Rahmenteil 6 und 7, Zugelemente 5 und 5' und Klemmen 10, analog der Darstellung aus Figur 4a. Die Zugelemente 5 und 5' sind mittels Spannvorrichtungen 25 an den Rahmenteilen 6 und 7 einstellbar befestigt. In Figur 9b verlaufen die Zugelemente 5 und 5' annähernd parallel neben einander und in Figur 9c verlaufen die Zugelemente 5 und 5' über Kreuz, wie es bei den Figuren 1a und 1 b genauer erläutert ist.

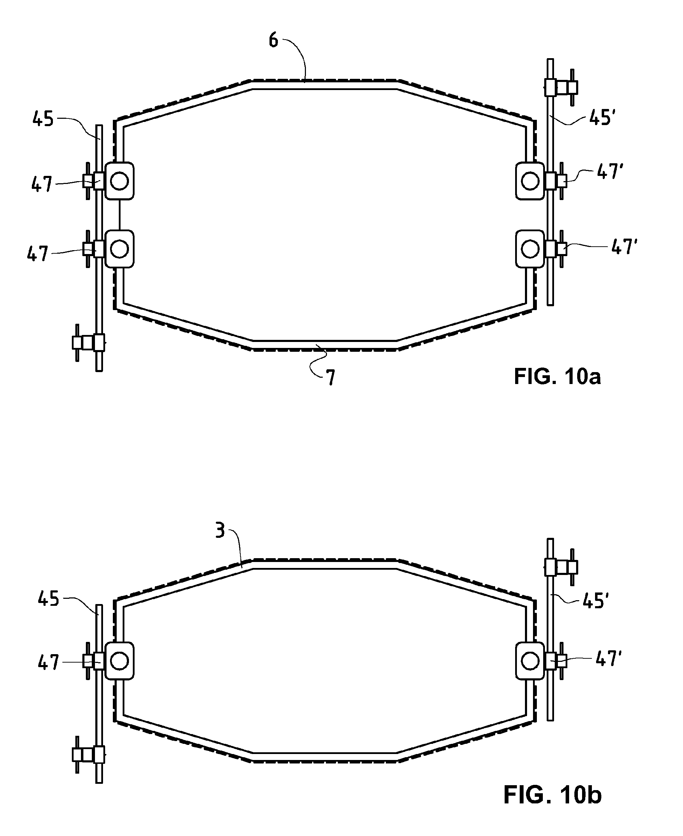

In den Figuren 10a und 10b sind schematische Beispiele für Halterungen zweier Varianten eines Rahmens einer Dehneinrichtung nach der Erfindung dargestellt. Die Halterung wird durch zwei sich gegenüberliegende Horizontalstreben 45, 45' gebildet, zwischen welchen die Dehneinrichtung gehalten wird. In Figur 10a ist eine Variante einer Dehneinrichtung mit zwei zueinander spiegelsymmetrisch angeordneten Rahmenteilen 6 und 7 gezeigt. Die Rahmenteile 6 und 7 werden jeweils mit einem Befestigungsmitteln 47 an der Horizontalstrebe 45 und mit einem Befestigungsmitteln 47' an der Horizontalstrebe 45' gehalten. Der Abstand der Rahmenteile 6 und 7 ist einstellbar, indem die Befestigungsmittel 47 und 47' entlang der Horizontalstreben 45 und 45' verschoben werden. In Figur 10b ist eine Variante einer Dehneinrichtung mit einem einteiligen Rahmen 3 gezeigt. Der Rahmen wird wiederum mittels Befestigungsmitteln 47 und 47' an den sich gegenüberliegenden Horizontalstreben 45 und 45' gehalten. Der Rahmen 3, bzw. die Rahmenteile 6 und 7 werden mittels den Horizontalstreben 45 und 45' über einer Bauchdeckenöffnung positioniert, so dass eine Dehneinrichtung mit Zugelementen, wie beispielsweise in den Figuren 9b und 9c gezeigt, eingerichtet werden kann.

**Bezugszeichen**

| | | | |
|---|---|---|---|
| 1 | Bauchdecke | 22 | Knopf |
| 2 | Öffnung | 23 | Karabiner |
| 3 | Rahmen | 24 | Skala |
| 4 | Haltemittel | 25 | Spannvorrichtung |
| 5, 5' | Zugelement | 26 | Haltearm |
| 6 | erstes Rahmenteil | 30, 30' | Haken |
| 7 | zweites Rahmenteil | 31 | Zahnstange |
| 8 | Haken | 32 | Träger |
| 9 | Stütze | 33 | Schraubhebel |
| 10 | Klemme | 40 | Operationstisch |
| 11 | Branchen | 41 | Traggestell |
| 12 | Verschluss | 42 | Auflage |
| 13 | Halmen | 43 | Patient |
| 14 | Schiene | 44 | Vertikalstrebe |
| 15 | Stange | 45, 45' | Horizontalstrebe |
| 16 | Einkerbung | 46 | Kloben |
| 17 | Klemmschlitz | 47, 47' | Befestigungsmittel |
| 18 | Backenklemme | | |
| 19 | Haltebacken | Z | Zugkraft |
| 20 | Anschlag | Zp | Parallelkomponente |
| 21 | Verdickung | | |

## Patentansprüche

1. Dehneinrichtung zum Dehnen einer Bauchdecke (1) an einer Öffnung (2) der Bauchdecke, die Zugelemente (5) mit einem Befestigungsmittel (8; 10) aufweist, mit dem ein Zugelement an einem Rand der Bauchdeckenöffnung (2) lösbar an der Bauchdecke (1) befestigbar ist, **dadurch gekennzeichnet, dass**
ein Rahmen (3) über der Öffnung (2) positionierbar ist und zum Halten der Zugelemente unter einer Zugkraft vorgesehen ist, wobei mehrere Zugelemente zueinander verteilt um den Rahmen vorgesehen sind, um die Zugkraft verteilt um den Rand der Öffnung auf die Bauchdecke (1) zu übertragen.

2. Dehneinrichtung nach Anspruch 1, **dadurch gekennzeichnet, dass** der Rahmen (3) Haltemittel aufweist, mit welchen die Zugelemente (5) unter einer Zugkraft am Rahmen (3) lösbar gehalten ist.

3. Dehneinrichtung nach Anspruch 2, **dadurch gekennzeichnet, dass** für jedes Zugelement (5) ein Haltemittel am Rahmen (3) vorgesehen ist.

4. Dehneinrichtung nach einem der vorhergehenden Ansprüche, **dadurch gekennzeichnet, dass** am Rahmen (3) wenigstens zwei gegenüberliegende Rahmenteile (6, 7) vorgesehen sind, die zum Anordnen an gegenüberliegenden Seiten der Öffnung (2) ausgebildet sind.

5. Dehneinrichtung nach Anspruch 4, **dadurch gekennzeichnet, dass** die zwei gegenüberliegenden Rahmenteile (6, 7) relativ zueinander beweglich sind.

6. Dehneinrichtung nach einem der vorhergehenden Ansprüche, **dadurch gekennzeichnet, dass** erste Zugelemente (5) an einem ersten Rahmenteil (6) und zweite Zugelemente (5') an einem zweiten, dem ersten gegenüberliegenden Rahmenteil (7) derart am Rahmen (3) gehalten sind, dass die ersten Zugelemente (5) und die zweiten Zugelemente (5') gekreuzt verlaufen.

7. Dehneinrichtung nach einem der vorhergehenden Ansprüche, **dadurch gekennzeichnet, dass** die Zugelemente (5) zumindest einen elastischen Bereich aufweisen und vorzugsweise überwiegend elastische ausgebildet sind.

8. Dehneinrichtung nach einem der vorhergehenden Ansprüche, **dadurch gekennzeichnet, dass** die Haltemittel als Klemmvorrichtung oder Schlitz (17) ausgebildet sind.

9. Dehneinrichtung nach einem der vorhergehenden Ansprüche, **dadurch gekennzeichnet, dass** ein Haltemittel eine Halterung (17; 19) aufweist, die zum Halten des Zugelements mit einem am Zugelement vorgesehenen Haltegegenstück (21) zusammenwirkt.

10. Dehneinrichtung nach einem der vorhergehenden Ansprüche, **dadurch gekennzeichnet, dass** am Rahmen (3) wenigstens eine Spannvorrichtung (25) zum Anlegen der Zugkraft an den Zugelementen (5) angeordnet ist.

11. Dehneinrichtung nach einem der vorhergehenden Ansprüche, **dadurch gekennzeichnet, dass** sie einen Kraftmesser zur Messung einer von einem Zugelement ausgeübten Zugkraft (Z) umfasst.

12. Dehneinrichtung nach einem der vorhergehenden Ansprüche, **dadurch gekennzeichnet, dass** sie einen Längenmesser zur Messung einer von einem Zugelement gezogenen Länge umfasst.

13. Dehneinrichtung nach einem der vorhergehenden Ansprüche, **dadurch gekennzeichnet, dass** sie einen Kraftbegrenzer zur Begrenzung einer von einem Zugelement ausgeübten Zugkraft umfasst.

14. Dehneinrichtung nach einem der vorhergehenden Ansprüche, **dadurch gekennzeichnet, dass** wenigstens eines der Zugelemente (5) eine Längenskala (24) entlang seiner Länge aufweist.

15. Dehneinrichtung nach einem der vorhergehenden Ansprüche, **dadurch gekennzeichnet, dass** ein Zugelement (5) an einem Endbereich am Rahmen (3) befestigbar ist und am anderen Endbereich ein Befestigungsmittel in Form einer Klemme (10) oder eines Hakens (8) zum Befestigen des Zugelements (5) an der Bauchdecke (1) aufweist.
